(19) **Europäisches Patentamt / European Patent Office / Office européen des brevets**

(11) **EP 2 451 809 B1**

(12) **EUROPEAN PATENT SPECIFICATION**

(45) Date of publication and mention
of the grant of the patent:
**12.10.2016 Bulletin 2016/41**

(21) Application number: **10732304.0**

(22) Date of filing: **09.07.2010**

(51) Int Cl.:
**C07D 453/02** (2006.01)

(86) International application number:
**PCT/EP2010/004204**

(87) International publication number:
**WO 2011/003624 (13.01.2011 Gazette 2011/02)**

(54) **A PROCESS FOR THE PREPARATION AND PURIFICATION OF SOLIFENACIN SALTS**

VERFAHREN ZUR HERSTELLUNG UND REINIGUNG VON SOLIFENACINSALZEN

PROCÉDÉ POUR LA PRÉPARATION ET LA PURIFICATION DE SELS DE SOLIFÉNACINE

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **09.07.2009 SI 200900188
15.07.2009 SI 200900193**

(43) Date of publication of application:
**16.05.2012 Bulletin 2012/20**

(73) Proprietor: **KRKA, D.D., Novo Mesto
8501 Novo mesto (SI)**

(72) Inventors:
 • **KLJAJIC, Alen
  3000 Celje (SI)**
 • **RUZIC, Milos
  3000 Celje (SI)**
 • **PRUDIC, Darja
  8000 Novo Mesto (SI)**
 • **PECAVAR, Anica
  8000 Novo Mesto (SI)**
 • **BUKOVEC, Polona
  8000 Novo Mesto (SI)**
 • **ZUPET, Rok
  1000 Ljubljana (SI)**

(74) Representative: **Andrae | Westendorp
Patentanwälte Partnerschaft
Uhlandstraße 2
80336 München (DE)**

(56) References cited:
 **WO-A1-2009/011844     WO-A2-2008/013851**

**Description**

**TECHNICAL FIELD**

The invention relates to a preparation and purification processes of solifenacin succinate, particularly to a crystallization process of solifenacin succinate, leading to high purity of the obtained product as well as to the desired average particle size and desired modality of the particle size distribution.

**BACKGROUND OF THE INVENTION**

Solifenacin, with its chemical name (1S)-(3R)-1-azabicyclo[2.2.2]oct-3-yl 3,4-dihydro-1-phenyl-2(1*H*)-isoquinoline carboxylate (I) is used for symptomatic treatment of urgent incontinence and/or increased frequency of urinating and urgency of urinating in patients with a hyperactive urinary bladder. The commercial tablet is marketed under the name Vesicare® and has been approved by the FDA for once daily treatment of OAB in 5 mg and 10 mg tablets. Each Vesicare tablet contains 5 or 10 mg of solifenacin succinate and is formulated for oral administration.

The technical problem to be solved was to develop the method to decrease the impurity content of solifenacin succinate, as well as the provision of a process yielding the selected average particle size and a mono-modal average particle size distribution, characterized in that the average particle size can easily be achieved by controlling the seeding temperature and the aging of the crystallization at the seeding temperature, $T_{seeding}$. There was a need to develop an efficient process with high yield and appropriate for industrial use.

Due to agglomeration and/or un-wanted nucleation processes present during the crystallization process a product, which possesses a bi- or even multi-modal average particle size distributions, can be prepared. For efficient formulation development of pharmaceutically active ingredients controlled modality and non-agglomerated particles with defined average size are particularly desired.

EP 1 714 965 A1 describes the purification of solifenacin succinate, solifenacin hydrochloride and solifenacin oxalate. In Reference Example 2, solifenacin hydrochloride is crystallized from a mixture of acetonitrile and ethyl ether, and in Reference Example 4 solifenacin oxalate is deposited from a mixture of isopropanol and isopropyl ether. The purity of both was found unsatisfactory compared to the purity of solifenacin succinate prepared according to Examples 2 and 3 wherein solifenacin succinate was dissolved in a mixture of ethanol and ethyl acetate with heating, subsequent addition of additional solvent mixture, cooling to 50°C, seeding the mixture at this temperature and cooling it to 0°C or 30°C, respectively. In the later case (Example 3), the reaction mixture was further heated to 50°C and finally cooled to 0°C over 5 hours. A means of controlling the average particle size and the modality of its distribution is not described.

WO 2008/011462 A2 enumerates a number of solvents suitable for the crystallization of solifenacin salts but specifically only uses acetone, ethanol and ethyl acetate for the preparation of solifenacin succinate (Example 9, Example 8). The mixture was either heated to 75°C and maintained for about 2.5 hours and then gradually cooled to 4°C and aged for about 4 hours, or heated to about 60°C and aged for about 1 hour, cooled to 12°C and maintained for about 1 hour. In the case of acetone, a second crystallization was needed. Example 10 describes the crystallization of solifenacin hydrochloride from acetonitrile and diethyl ether. No seeding nor any other means of controlling the average particle size and the modality of its distribution is described.

WO 2008/013851 teaches the preparation of crystalline solifenacin succinate from various solvents i.a. from $C_1$-$C_4$ alcohols, or mixtures comprising a $C_1$-$C_4$ alcohol. With the exception of 95% ethanol no reaction mixture comprising water is disclosed by WO 2008/013851. Also here, no seeding by any means of controlling the average particle size and the modality of its distribution is described.

DESCRIPTION OF THE FIGURES

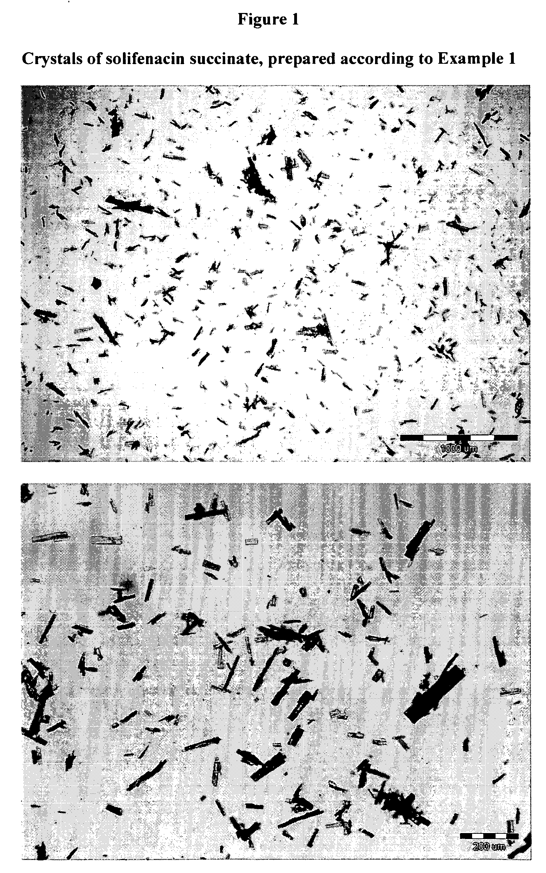

Fig. 1 shows two photographs of solifenacin succinate crystals, prepared according to Example 1.

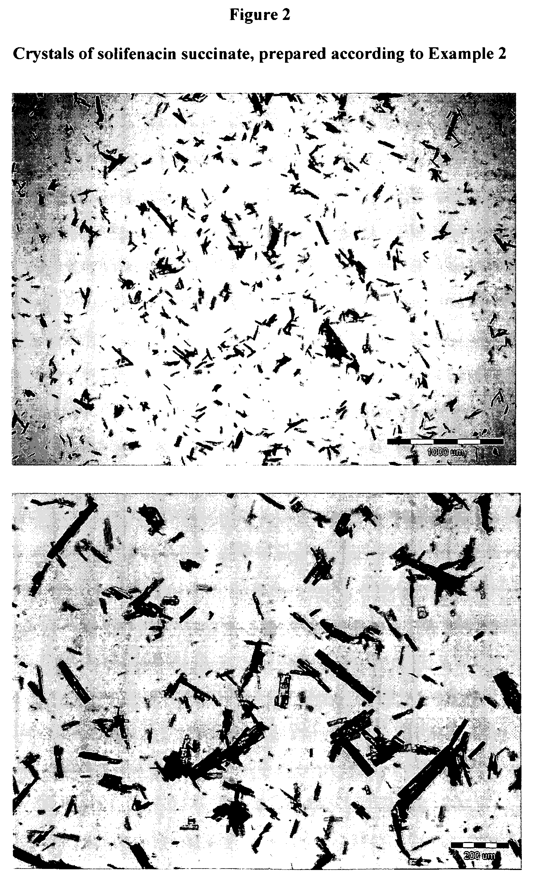

Fig. 2 shows two photographs of solifenacin succinate crystals, prepared according to Example 2.

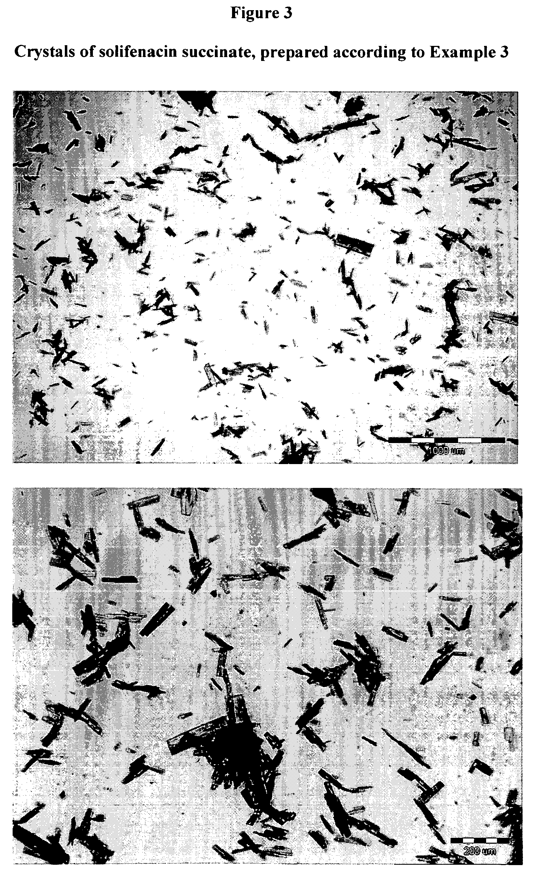

Fig. 3 shows two photographs of solifenacin succinate crystals, prepared according to Example 3.

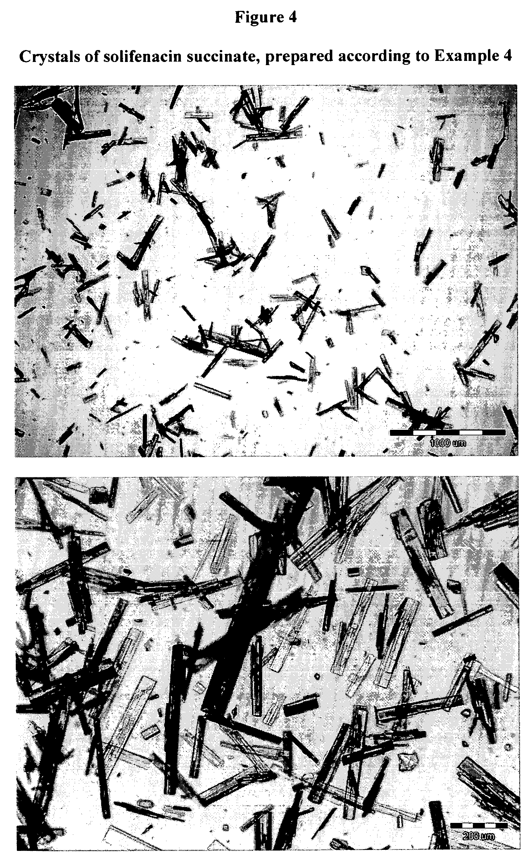

Fig. 4 shows two photographs of solifenacin succinate crystals, prepared according to Example 4.

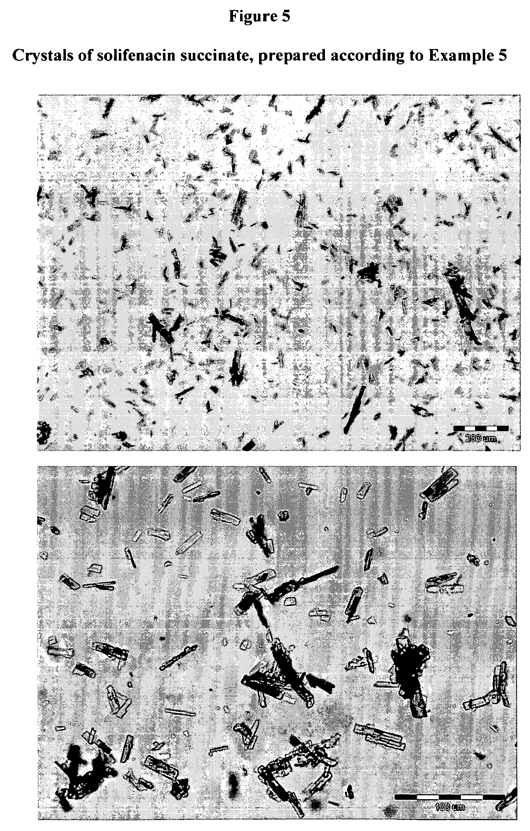

Fig. 5 shows two photographs of solifenacin succinate crystals, prepared according to Example 5.

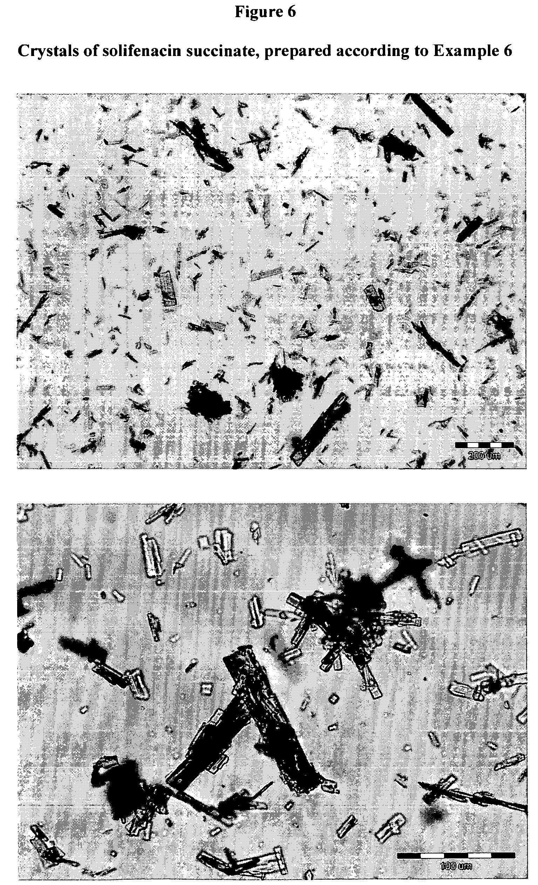

Fig. 6 shows two photographs of solifenacin succinate crystals, prepared according to Example 6.

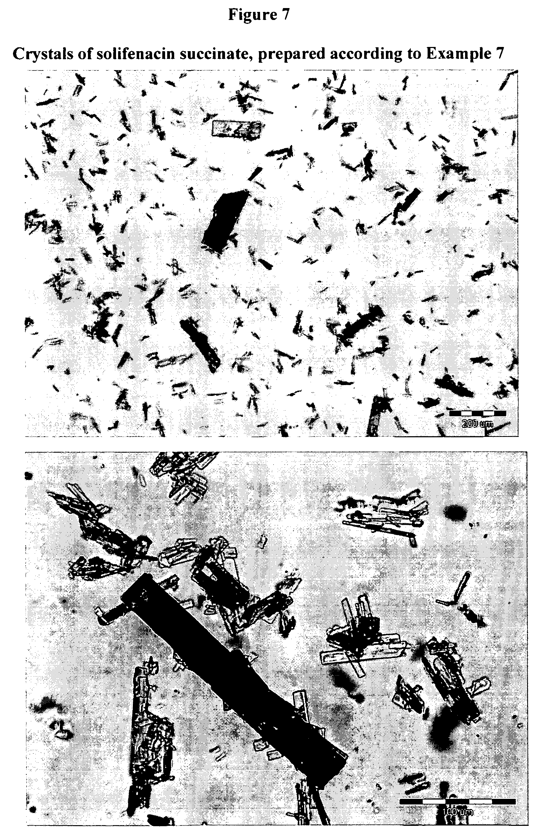

Fig. 7 shows two photographs of solifenacin succinate crystals, prepared according to Example 7.

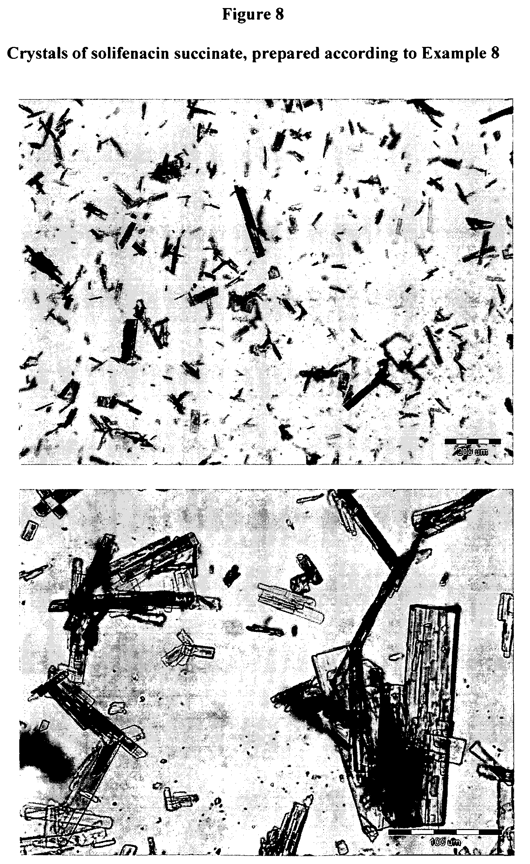

Fig. 8 shows two photographs of solifenacin succinate crystals, prepared according to Example 8.

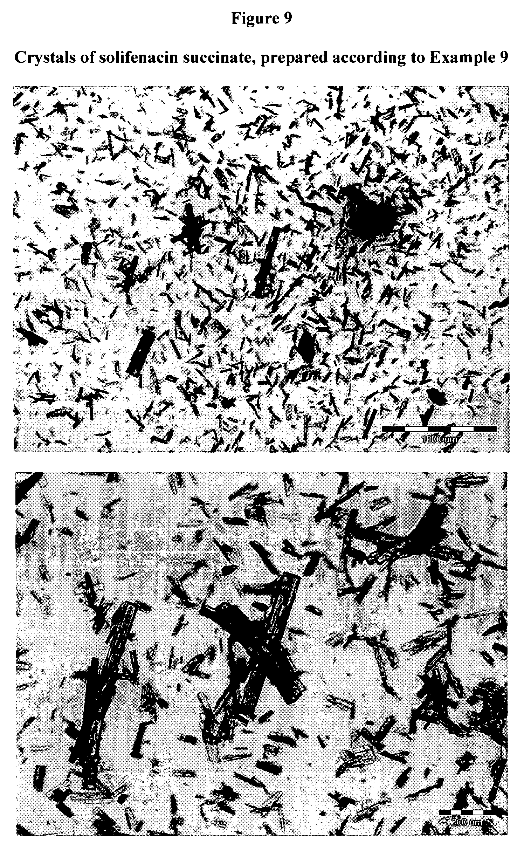

Fig. 9 shows two photographs of solifenacin succinate crystals, prepared according to Example 9.

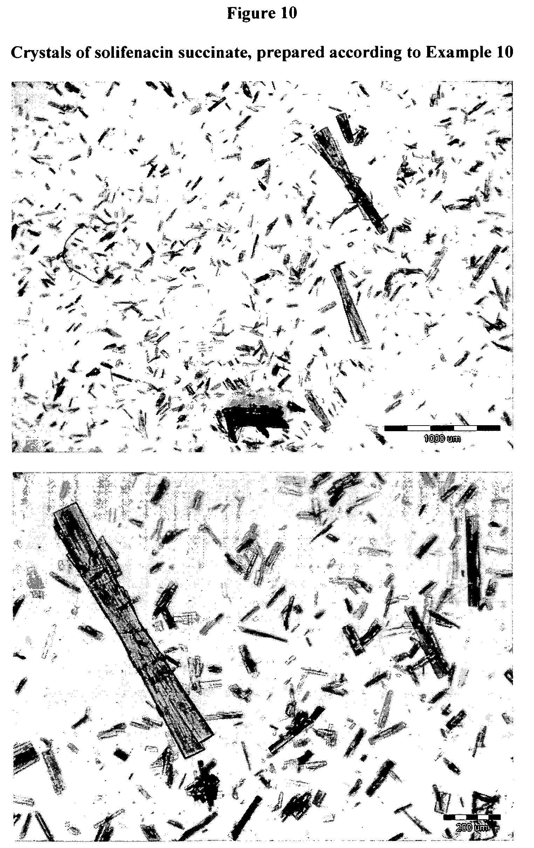

Fig. 10 shows two photographs of solifenacin succinate crystals, prepared according to Example 10.

Fig.s 11 to 20 show particle size distributions of products prepared according to Examples 1 to 10, respectively.

## DESCRIPTION OF THE INVENTION

According to an aspect, the present invention provides a crystallization process as defined in claim 1 annexed.
According to the invention there is provided a crystallization process for the preparation and/or purification of solifenacin succinate applying $C_5$-$C_{10}$ alcohols comprising water. Preferably, solifenacin succinate is crystallized from a mixture comprising pentanol and water or from a solvent mixture comprising hexanol and water.
According to the invention there it is further provided:

1.) a process for the crystallization of a solifenacin salt, which is solifenacin succinate, characterized in that:

i) A solifenacin salt, which is solifenacin succinate, is dissolved in a solvent mixture comprising water and a $C_5$ to $C_{10}$ alcohol, preferably n-pentanol, n-hexanol, or n-heptanol, most preferably n-hexanol, at a temperature between 40°C and 150°C, preferably between 75°C and 100°C until all solifenacin salt dissolves,
ii) optionally cooling the dissolved mixture to the seeding temperature $T_{seeding}$, which is between 40°C and 80°C, preferably between 50°C and 70°C, most preferably between 50°C and 60°C,
iii) optionally seeding the dissolved mixture with crystals of solifenacin salt,
iv) optionally aging the seeded media to additionally control the nucleation process for 10 to 120 minutes,
v) cooling the mixture to a final temperature between -40 °C and 40 °C, preferably to a temperature between 5°C and 10°C in a time between 0.5 and 10 hours, preferably in between 2 and 3 hours, and maintained at the final temperature for 15 minutes to 20 hours if needed,
vi) optionally filtering and optionally washing the crystals obtained; and

2.) a process for the preparation of a solifenacin salt, which is solifenacin succinate, using solifenacin base and a counter acid for the salt preparation, preferably succinic acid characterized in that:

vii) solifenacin base, is dissolved in a solvent mixture comprising a $C_5$ to $C_{10}$ alcohol and optionally comprising water, preferably n-pentanol, n-hexanol, or n-heptanol, most preferably n-hexanol, at a any given temperature,
viii) addition of the counter acid, which can be in concentrated form (solid, gas or liquid) or separately dissolved or suspended in any (anti)solvent,
ix) optionally seeding the dissolved mixture with crystals of solifenacin salt at the seeding temperature $T_{seeding}$ between 40°C and 80°C, preferably between 50°C and 70°C, most preferably between 50°C and 60°C,
x) optionally aging the seeded media,
xi) cooling the mixture to a final temperature between -40 °C and 40 °C, in a time between 0.5 and 10 hours, preferably in between 2 and 3 hours, and maintaining it at the final temperature for 15 minutes to 20 hours if needed,
xii) optionally filtering and optionally washing the crystals obtained.

The average particle size of formed product can be controlled in a crystallization process with seeding and the degree of super-saturation applied through the crystallization process which consequently influences the rate and type of nucleation and the rate of crystal growth through the crystallization process.
It was observed by the authors of this invention that in the case of solifenacin succinate, the cooling gradient parameter (cooling ramp), solvent concentration and temperature of spontaneous or secondary nucleation can be used for control of average particle size and modality of its distribution and also the purity of prepared material. Surprisingly it was found out, that aging the media after seeds are added can have crucial role in effective preparation of solifenacin succinate with one-modal particle size distribution on a large-scale production, especially if optimized seeding temperature is used. It has been discovered by the present invention that an efficient control of average particle size, modality and purity of solifenacin succinate, is achieved by selecting the seeding temperature from the range of 40°C to 80°C, preferably 50°C to 70°C, most preferably 50°C to 60°C, the time of aging the seeded mixture from 0 to 250 minutes, preferably from 10 to 120 minutes, and the cooling ramp between 0.1 K/min to 1.5 K/min, preferably between 0.2 K/min and 0.5 K/min.

The term "particle size distribution" means a particle size frequency distribution that shows the percentage of particles (normalized in means of mass, volume or count) found in each size range and usually displayed in a histogram form. The term »unimodal distribution« or »one-modal distribution« refers to one whose histogram has a single peak, so that the frequencies at first increase and then decrease. The term »bi-modal distribution« is one whose histogram has two peaks. Similarly, there can be trimodal distributions and so on.

The term "dried product" means a solifenacine salt with solvent/anti-solvent content below 0.50 w/w %. This refers to mass percentage content of $C_5$ to $C_{10}$ alcohol and water present in dried product.

The term "applying $C_5$-$C_{10}$ alcohols" as used in the present application encompasses applying or using at least one $C_5$-$C_{10}$ alcohol. The term "applying a mixture of $C_5$-$C_{10}$ alcohols and water" as used in the present application encompasses applying or using a mixture of at least one $C_5$-$C_{10}$ alcohol and water.

The $C_5$ to $C_{10}$ alcohol(s) applied in a crystallization process for the preparation and/or purification of solifenacin salts, can be at least one alcohol selected from the group of $C_5$ alcohols, $C_6$ alcohols, $C_7$ alcohols, $C_8$ alcohols, $C_9$ alcohols, and $C_{10}$ alcohols, preferably at least one alcohol selected from the group of n-pentanol, isopentanol, sec-pentanol, n-hexanol, iso hexanol, sec-hexanol, n-heptanol, n-octanol, n-nonanol and n-decanol, preferably n-pentanol, n-hexanol, or n-heptanol, most preferably n-hexanol. In particular, a mixture comprising one or more of these $C_5$ to $C_{10}$ alcohol(s) and water can be applied. The crystallization process for the preparation and/or purification of solifenacin succinate can comprise the step of dissolving a solifenacin salt, which is solifenacin succinate, in particular the step of dissolving a solifenacin salt, which is solifenacin succinate, in a mixture comprising at least one $C_5$-$C_{10}$ alcohol and water.

According to one embodiment of the invention, a mixture of at least one $C_5$-$C_{10}$ alcohol and at least one further alcohol having less than 5 or more than 10 carbon atoms can be applied or used, with water.

The volume ratio between water (preferably purified and/or degassed water) to an organic solvent is in particular between 0.1% and 10%, preferably between 0.4% and 5%, most preferably between 0.5 and 3 %(V/V %).

The mass to volume ratio between solifenacin succinate and the solvent mixture applied is in particular between 1/50 to 1/1 kg/L, preferably between 1/10 to 1/1.5 kg/L and most preferably between 1/5 to 1/2 kg/L.

If n-hexanol is applied, the mass to volume ratio between solifenacin succinate, and the solvent mixture applied is in particular between 1 kg solifenacin succinate to 2 L of n-hexanol and water and I kg solifenacin succinate to 5 L of n-hexanol and water, preferably between 1 kg solifenacin succinate to 3 L of n-hexanol and water and 1 kg solifenacin succinate to 4 L of n-hexanol and water. The volume to volume ratio between water and n-hexanol in case of solifenacin succinate is preferably between 1 to 50 and I to 200, preferably between 1 to 70 and 1 to 150, most preferably between 1 to 90 and 1 to 120.

The crude solifenacin succinate according to the present invention when wet may still comprise a water/$C_5$-$C_{10}$ alcohol content of 80 % w/w to below 1 % w/w directly from the filtration/centrifugation step.

The average particle size of the obtained solifenacin succinate may be optimized to the average diameter of 20 to 600 micrometers ($\mu$m), preferably to the average particle size from 30 - 400 micrometers, most preferably to the average particle size between 40 and 250 micrometers by the process of the present invention. The morphology of particles formed, needle or/to plate type, are presented in the Figures. Particle size was measured using a Malvern-Mastersizer 2000 equipped with Hydro S dispersion unit. Vegetable oil, in particular corn oil, was used as the dispersion medium, no sonnication was applied. Absorption value was set to 1 (A=1).

The term "average particle size" as used herein refers to the volume mean diameter of particles.

The term "$d_{90}$ value" means that at least 90% of the particles have volume diameter of less than the specified value. The same approach goes for the $d_{50}$ and $d_{10}$.

When there is the need for special particle size, particularly average particle size between 2 and 40 micrometers, preferably between 10 and 25 micrometers, most preferably between 15 and 20 micrometers, prior to its isolation the particles of solifenacin succinate may be optimized in size with the use of a dispergator. As the dispergator any commercially available product may be used, such as Ultra-TurraX®. Another option is the use of mills after the isolation of solifenacin succinate. During milling the mechanical force exerted on the particle surface leads to particle size reduction. Milling can be performed by any milling process known in the art, for example using a ball mill (planetary ball mill or mixer mill), hammer mill, bead mill, disc mill, ultrasonic mill, torus mill, impact mill, vibration mill, pin mill or air jet mill. The basic principle of treatment in an air jet mill is collision and attrition between particles suspended within the high velocity air stream which introduces the power to the milling chamber. In a ball mill, particles are fractured by impact of grinding media (e.g. balls, cubes, cylinders, jars etc.) that can occupy up to half of the mill chamber volume. Due to rotation of the chamber the grinding media falls from an elevated position. Friction is also present among all elements, contributing significantly to the attrition and consequently to the amorphous nature of the material being milled. One of the most widely used mills in the pharmaceutical industry is the hammer mill. In such equipment, particles are exposed to the impact of rapidly rotating hammers. During milling, material may additionally hit the perforated screen that is placed over the chamber outlet.

The obtained crystalline form of solifenacin succinate is preferably Form I, characterized by powder X-ray diffraction (PXRD) pattern having peaks at about: 3.7, 5.4, 7.4, 8.2, 9.4, 11.1, 11.8, 12.5, 13.5, 14.1, 14.4, 15.5, 16.4, 17.1.17.6, 18.1, 18.6, 19.1, 19.6, 20.0, 20.8, 21.2, 21.8, 22.8, 23.2, 23.8, 24.7, 25.2, 25.6, 26.2, 26.5, 26.8, 27.5, 28.2, 28.5, 29.0, 29.5, 30.2 $\pm$ 0,2 2-theta degrees or 3.7, 7.4, 11.1, 11.9, 13.5, 14.1, 14.8, 15.5, 15.8, 17.6, 18.1, 18.5, 19.3, 20.2, 21.0, 21.8, 22.3, 22.7, 23.0, 23.2, 23.9, 25.1, 25.8, 26.1, 26.6, 27.2, 27.6, 28.6, 29.5 $\pm$ 0,2 2-theta degrees, as measured on Panalytical x'pert pro diffraktometer with CuK$\alpha$ source.

The purity and the enantiomeric purity of solifenacin succinate, is determined with the following HPLC methods:

**Column:** Ascentis Express C8, 2.7 $\mu$m particles, 100 x 4.6 mm i.d.,
**Eluent A:** 0.02M NaH$_2$PO$_4$, pH adjusted to 2.5 with phosphoric acid
**Eluent B:** acetonitrile
**Eluent C:** methanol
**Gradient:**

| Time (min) | % A | %B | % C |
|---|---|---|---|
| 0 | 75 | 12.5 | 12.5 |
| 3.5 | 75 | 12.5 | 12.5 |
| 16.5 | 0 | 50 | 50 |
| 18.5 | 0 | 50 | 50 |
| 20 | 0 | 87.5 | 12.5 |
| 23 | 0 | 87.5 | 12.5 |
| 25 | 75 | 12.5 | 12.5 |

**Post time:** 4 min
**Flow-rate:** 0.7 ml/min
**Detection:** UV, 210 nm
**Injection volume:** 5 $\mu$l
**Column temperature:** 25°C
**Diluent:** methanol

**Sample preparation:**

Accurately weigh about 20 mg of sample into 25 ml volumetric flask, dissolve and dilute to volume with diluent.

**Calculation:**

Use area per cent method. Do not integrate solvent peaks.

**SOLIFENACIN -enantiomeric purity:**

**Column:** Chiralpak IA, 5 $\mu$m particles, 250 x 4.6 mm i.d.,
**Eluent A:** hexan
**Eluent B:** ethanol/trifluoroacetic acid/diethylamine in the ratio 99/0.5/0.5
**Mobile phase:** eluent A : eluent B in the ratio 30 : 70
**Flow-rate:** 0.6 ml/min
**Detection:** UV, 215 nm
**Injection volume:** 20 $\mu$l
**Column temperature:** 25°C
**Diluent:** ethanol

**Sample preparation:**

Accurately weigh about 20 mg of sample into 20 ml volumetric flask, dissolve and dilute to volume with diluent.

**Calculation:**

$$\% \text{ S-enantiomer} = \frac{\text{area}_{\text{S-enantiomer}}}{\sum(\text{area}_{\text{S-enantiomer}} + \text{area}_{\text{R-enantiomer}})} \times 100$$

Disclosed herein is:

1.) A crystallization process for the preparation and/or purification of solifenacin succinate, applying $C_5$-$C_{10}$ alcohols.
2.) A process according to item 1, characterized in that the crystallization process for the preparation and/or purification of solifenacin succinate, applies a mixture of $C_5$-$C_{10}$ alcohols and water.
3.) A process according to items 1 and 2, characterized in that the alcohol is chosen from the group of n-pentanol and n-hexanol.
4.) A process according to items 1 and 2, characterized in that:

   i.) Solifenacin succinate, is dissolved in a solvent mixture comprising water and a $C_5$ to $C_{10}$ alcohol, preferably n-pentanol, n-hexanol, or n-heptanol, most preferably n-hexanol, at a temperature between 40°C and 150°C, preferably between 75°C and 100°C until all solifenacin salt dissolves,
   ii.) cooling the dissolved mixture to the seeding temperature $T_{\text{seeding}}$, which is between 40°C and 80°C, preferably between 50°C and 70°C, most preferably between 50°C and 60°C,
   iii.) optionally seeding the dissolved mixture with crystals of solifenacin succinate,
   iv.) optionally aging the seeded media to additionally control the nucleation process for 10 to 120 minutes,
   v.) cooling the mixture to a final temperature between -40 °C and 40 °C, preferably to a temperature between 5°C and 10°C in a time between 0.5 and 10 hours, preferably in between 2 and 3 hours, and maintained at the final temperature for 15 minutes to 20 hours if needed,
   vi.) filtering and washing the crystals obtained.

5.) A process according to items 1 and 2, characterized in that:

   i.) solifenacin base, is dissolved in a solvent mixture comprising a $C_5$ to $C_{10}$ alcohol and optionally comprising water, preferably n-pentanol, n-hexanol, or n-heptanol, most preferably n-hexanol, at a any given temperature,
   ii.) addition of the counter acid, which can be in concentrated form (solid, gas or liquid) or separately dissolved or suspended in any (anti)solvent,
   iii.) optionally seeding the dissolved mixture with crystals of solifenacin succinate at the seeding temperature $T_{\text{seeding}}$ between 40°C and 80°C, preferably between 50°C and 70°C, most preferably between 50°C and 60°C,
   iv.) optionally aging the seeded media,
   v.) cooling the mixture to a final temperature between -40 °C and 40 °C, in a time between 0.5 and 10 hours, preferably in between 2 and 3 hours, and maintaining it at the final temperature for 15 minutes to 20 hours if needed,
   vi.) filtering and washing the crystals obtained.

6.) A process according to any of items 1 to 5, characterized in that the cooling ramp to the final temperature is between 0.1 K/min to 1.5 K/min, preferably between 0.2 K/min and 0.5 K/min.
7.) A process according to any of items 1 to 6, characterized in that the mass to volume ratio between solifenacin salt, preferably solifenacin succinate, and the solvent mixture applied is between 1/50 to 1/1 kg/L, preferably between 1/10 to 1/1.5 kg/L and most preferably between 1/5 to 1/2 kg/L.
8.) A process according to any items 1 to 7, characterized in that after the supersaturated media is seeded, the aging at the temperature of seeding is applied.
9.) A process according to any items 1 to 8, characterized in that the temperature of seeding is below 65 °C.
10.) A crystallization process for solifenacin salt, using alcohols or alcohol/water mixtures characterized in seeding and/or aging below 65 °C.

11.) A process according to any items 1 to 9, characterized in that material with one-modal particle size distribution is obtained.

12.) A process according to any items 1 to 10, characterized in that material with one-modal particle size distribution and average particle size between 40 - 250 $\mu$m is obtained.

The present invention is illustrated by the following Examples without being limited thereto.

In the Examples, the Reactor Temperature (RT) versus time during the cooling process is presented as a linear function, but is not limited thereto. It is used as an average cooling rate for describing the time needed for cooling the mixture in the reactor from starting temperature to the desired (final) temperature. The real function of the reactor temperature versus time of crystallization process can be represented by any other continuous function.

The "temperature of seeding" ($T_{seeding}$) is the temperature, at which the seeds of pure chemical substance are added.

**Example 1**

A mixture comprising 80 ml of 1-hexanol and 0.8 ml of purified water was prepared. 20.0 g of solifenacin succinate was added and the formed suspension was heated to 85 °C (Reactor Temperature, RT) and maintained at this temperature for the next 30 minutes for all solid to dissolve. Continuous homogenization with mechanical mixer was applied during the crystallization process. Formed clear solution was cooled to 50 °C ($T_{seeding}$) in 30 minutes. 200 mg of seeds of pure solifenacin were added at the temperature of 50°C and maintained at this temperature for next 60 minutes. Then the formed suspension was cooled with average cooling ramp 0.22 K/min to 10 °C (in 180 minutes) and maintained at this temperature for the next 30 minutes for the crystallization to complete. Finally, the formed product was isolated with filtration and washed with 15 ml of 1-hexanol, cooled to 10 °C.

The isolated product was dried in a vacuum drier at 60 °C with a nitrogen flow applied. 18.4 g of dried product was obtained.

Yield = 92%

Average particle size: 83 $\mu$m

Particle size distribution:

| $D_{10}$ | 13.7 $\mu$m |
|---|---|
| $D_{50}$ | 54.7 $\mu$m |
| $D_{90}$ | 187.9 $\mu$m |

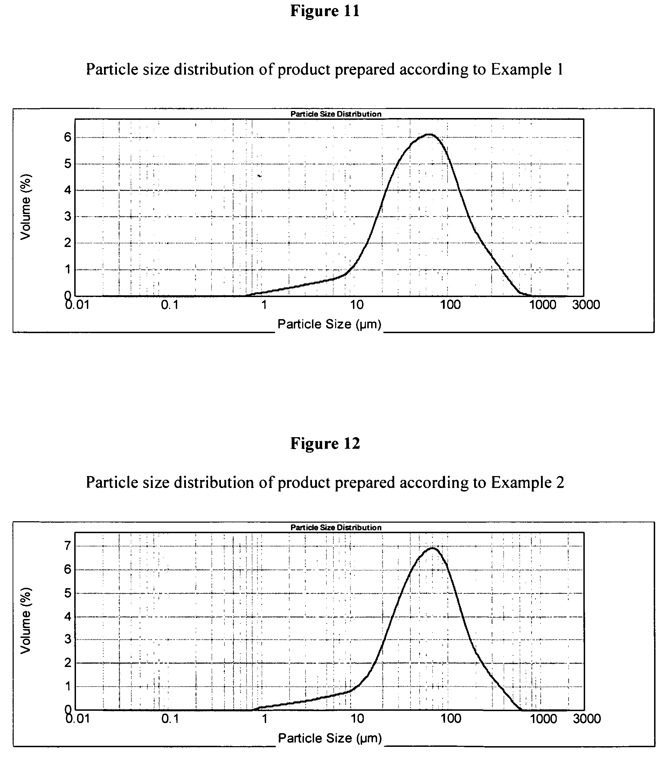

Particle size distribution of product prepared according to Example 1 is shown in Fig. 11.

**Example 2**

A mixture comprising 80 ml of 1-hexanol and 0.8 ml of purified water was prepared. 20.0 g of solifenacin succinate was added and the formed suspension was heated to 85 °C (RT) and maintained at this temperature for the next 30 minutes for all solid to dissolve. Continuous homogenization with mechanical mixer was applied during the crystallization process. Formed clear solution was cooled to 55 °C ($T_{seeding}$) in the next 30 minutes. 200 mg of seeds of pure solifenacin were added at the temperature of 55°C and maintained at this temperature for next 60 minutes. Then the formed suspension was cooled with average cooling ramp 0.25 K/min to 10 °C (in 180 minutes) and maintained at this temperature for the next 30 minutes for the crystallization to complete. Finally, the formed product was isolated with filtration and washed with 15 ml of 1-hexanol, cooled to 10 °C.

The isolated product was dried in a vacuum drier at 60 °C with a nitrogen flow applied. 18.4 g of dried product was obtained.

Yield = 92%

Average particle size: 85 $\mu$m

Particle size distribution:

| $D_{10}$ | 15.6 $\mu$m |
|---|---|
| $D_{50}$ | 60.2 $\mu$m |
| $D_{90}$ | 181.9 $\mu$m |

Particle size distribution of product prepared according to Example 2 is shown in Fig. 12.

### Example 3

A mixture comprising 80 ml of 1-hexanol and 0.8 ml of purified water was prepared in glass reactor. 20.0 g of solifenacin succinate was added and the formed suspension was heated to 85 °C (RT) and maintained at this temperature for the next 30 minutes for all solid to dissolve. Continuous homogenization with mechanical mixer was applied during the crystallization process. Formed clear solution was cooled to 60 °C ($T_{seeding}$) in 30 minutes. 200 mg of seeds of pure solifenacin were added at the temperature of 60°C and maintained at this temperature for next 60 minutes. Then the formed suspension was cooled with average cooling ramp 0.28 K/min to 10 °C (in 180 minutes) and maintained at this temperature for the next 30 minutes for the crystallization to complete. Finally, the formed product was isolated with filtration and washed with 15 ml of 1-hexanol, cooled to 10 °C.

The isolated product was dried in a vacuum drier at 60 °C with a nitrogen flow applied. 17.8 g of dried product was obtained.

Yield = 89%

Average particle size: 113 $\mu$m

Particle size distribution:

| | |
|---|---|
| $D_{50}$ | 20.4 $\mu$m |
| $D_{50}$ | 78.0 $\mu$m |
| $D_{90}$ | 243.5 $\mu$m |

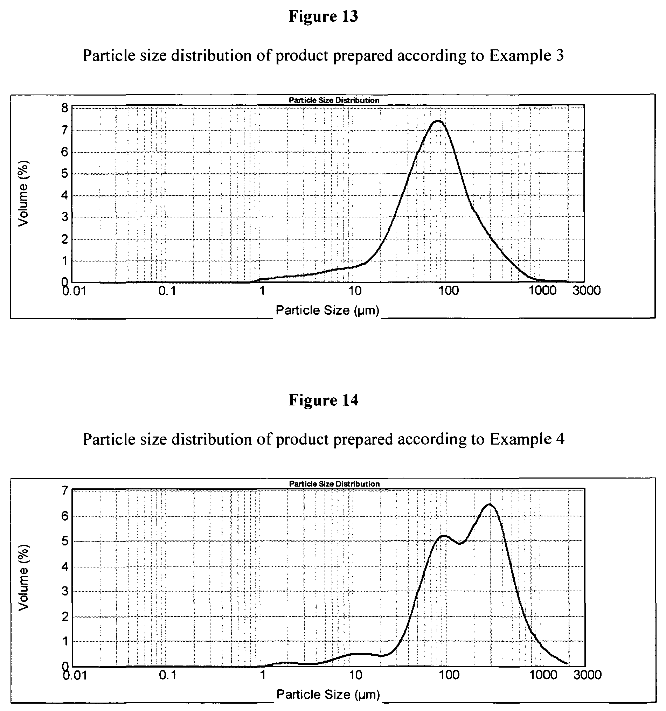

Particle size distribution of product prepared according to Example 3 is shown in Fig. 13.

### Example 4

A mixture comprising 80 ml of 1-hexanol and 0.8 ml of purified water was prepared in glass reactor. 20.0 g of solifenacin succinate was added and the formed suspension was heated to 85 °C (RT) and maintained at this temperature for the next 30 minutes for all solid to dissolve. Continuous homogenization with mechanical mixer was applied during the crystallization process. Formed clear solution was cooled to 65 °C ($T_{seeding}$) in 30 minutes. 200 mg of seeds of pure solifenacin were added at the temperature of 65°C and maintained at this temperature for next 60 minutes. Then the formed suspension was cooled with average cooling ramp 0.31 K/min to 10 °C (in 180 minutes) and maintained at this temperature for the next 30 minutes for the crystallization to complete. Finally, the formed product was isolated with filtration and washed with 15 ml of 1-hexanol, cooled to 10 °C.

The isolated product was dried in a vacuum drier at 60 °C with a nitrogen flow applied. 17.7 g of dried product was obtained.

Yield = 89%

Average particle size: 249 $\mu$m

Particle size distribution:

| | |
|---|---|
| $D_{10}$ | 46.4 $\mu$m |
| $D_{50}$ | 178.8 $\mu$m |
| $D_{90}$ | 529.5 $\mu$m |

Particle size distribution of product prepared according to Example 4 is shown in Fig. 14.

### Example 5

A mixture comprising 80 ml of 1-hexanol and 0.8 ml of purified water was prepared. 20.0 g of solifenacin succinate was added and the formed suspension was heated to 85 °C (RT) and maintained at this temperature for the next 30 minutes for all solid to dissolve. Continuous homogenization with mechanical mixer was applied during the crystallization process. Formed clear solution was cooled to 50 °C ($T_{seeding}$) in 30 minutes. 200 mg of seeds of pure solifenacin were added at the temperature of 50°. After that the suspension was immediately cooled with average cooling ramp 0.22 K/min to 10 °C (in 180 minutes) and maintained at this temperature for the next 30 minutes for the crystallization to complete. Finally, the formed product was isolated with filtration and washed with 15 ml of 1-hexanol, cooled to 10 °C.

The isolated product was dried in a vacuum drier at 60 °C with a nitrogen flow applied. 18.2 g of dried product was obtained.

Yield = 91%

Average particle size: 62 $\mu$m

Particle size distribution:

| | |
|---|---|
| $D_{10}$ | 7.7 $\mu$m |
| $D_{50}$ | 31.2 $\mu$m |
| $D_{90}$ | 156.6 $\mu$m |

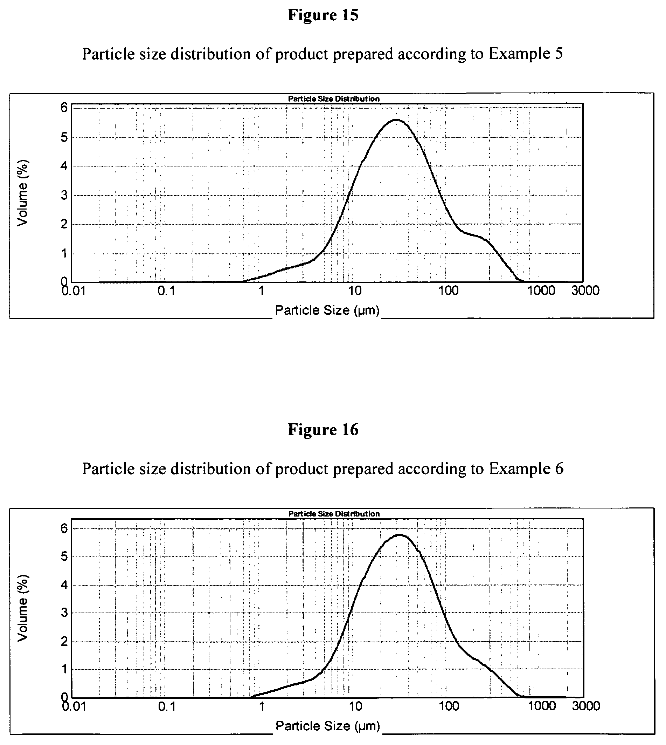

Particle size distribution of product prepared according to Example 5 is shown in Fig. 15.

**Example 6**

A mixture comprising 80 ml of 1-hexanol and 0.8 ml of purified water was prepared. 20.0 g of solifenacin succinate was added and the formed suspension was heated to 85 °C (RT) and maintained at this temperature for the next 30 minutes for all solid to dissolve. Continuous homogenization with mechanical mixer was applied during the crystallization process. Formed clear solution was cooled to 55 °C ($T_{seeding}$) in the next 30 minutes. 200 mg of seeds of pure solifenacin were added at the temperature of 55°. After that the suspension was immediately cooled with average cooling ramp 0.25 K/min to 10 °C (in 180 minutes) and maintained at this temperature for the next 30 minutes for the crystallization to complete. Finally, the formed product was isolated with filtration and washed with 15 ml of 1-hexanol, cooled to 10 °C.

The isolated product was dried in a vacuum drier at 60 °C with a nitrogen flow applied. 18.2 g of dried product was obtained.

Yield = 91%

Average particle size: 57 $\mu$m

Particle size distribution:

| | |
|---|---|
| $D_{10}$ | 8.2 $\mu$m |
| $D_{50}$ | 31.7 $\mu$m |
| $D_{90}$ | 132.6 $\mu$m |

Particle size distribution of product prepared according to Example 6 is shown in Fig. 16.

**Example 7**

A mixture comprising 80 ml of 1-hexanol and 0.8 ml of purified water was prepared. 20.0 g of solifenacin succinate was added and the formed suspension was heated to 85 °C (RT) and maintained at this temperature for the next 30 minutes for all solid to dissolve. Continuous homogenization with mechanical mixer was applied during the crystallization process. Formed clear solution was cooled to 60 °C ($T_{seeding}$) in the next 30 minutes. 200 mg of seeds of pure solifenacin were added at the temperature of 60°. After that the suspension was immediately cooled with average cooling ramp 0.28 K/min to 10 °C (in 180 minutes) and maintained at this temperature for the next 30 minutes for the crystallization to complete. Finally, the formed product was isolated with filtration and washed with 15 ml of 1-hexanol, cooled to 10 °C.

The isolated product was dried in a vacuum drier at 60 °C with a nitrogen flow applied. 18.5 g of dried product was obtained.

Yield = 93%

Average particle size: 71 $\mu$m

Particle size distribution:

| | |
|---|---|
| $D_{50}$ | 9.4 $\mu$m |
| $D_{50}$ | 38.4 $\mu$m |

(continued)

| | |
|---|---|
| $D_{90}$ | 178.2 μm |

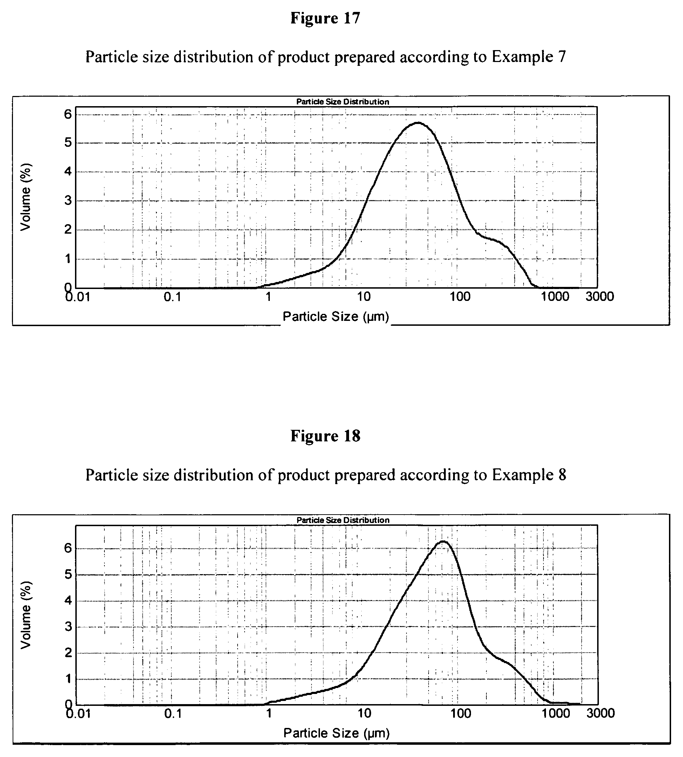

Particle size distribution of product prepared according to Example 7 is shown in Fig. 17.

**Example 8**

A mixture comprising 80 ml of 1-hexanol and 0.8 ml of purified water was prepared. 20.0 g of solifenacin succinate was added and the formed suspension was heated to 85 °C (RT) and maintained at this temperature for the next 30 minutes for all solid to dissolve. Continuous homogenization with mechanical mixer was applied during the crystallization process. Formed clear solution was cooled to 65 °C ($T_{seeding}$) in the next 30 minutes. 200 mg of seeds of pure solifenacin were added at the temperature of 65°. After that the suspension was immediately cooled with average cooling ramp 0.31 K/min to 10 °C (in 180 minutes) and maintained at this temperature for the next 30 minutes for the crystallization to complete. Finally, the formed product was isolated with filtration and washed with 15 ml of 1-hexanol, cooled to 10 °C.
The isolated product was dried in a vacuum drier at 60 °C with a nitrogen flow applied. 18.7 g of dried product was obtained.
Yield = 94%
Average particle size: 99 μm
Particle size distribution:

| | |
|---|---|
| $D_{10}$ | 12.9 μm |
| $D_{50}$ | 58.5 μm |
| $D_{90}$ | 231.7 μm |

Particle size distribution of product prepared according to Example 8 is shown in Fig. 18.

**Example 9**

**Prepation of seeding crystals**

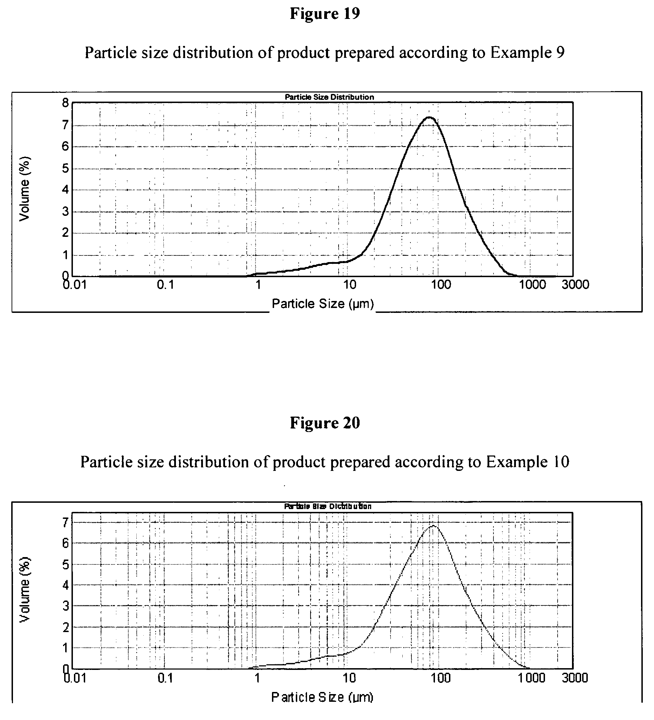

A mixture comprising 20 ml of 1-hexanol and 0.2 ml of purified water was prepared. 5.0 g of solifenacin succinate, prepared in Example 13, was added and the formed suspension was heated to 85 °C (RT) and maintained at this temperature for the next 30 minutes for all solid to dissolve. Continuous homogenization with mechanical mixer was applied during the crystallization process. Formed clear solution was cooled to 10 °C (RT) in the next 250 minutes with average cooling ramp 0.30 K/min and maintained at this temperature for the next 10 minutes for the crystallization to complete. Finally, the formed product was isolated with filtration and washed with 3 ml of 1-hexanol, cooled to 10 °C.
The isolated product was dried in a vacuum drier at 60 °C with a nitrogen flow applied.
Yield = 90 %
4.5 g of dried product was obtained.
Average particle size: 96 μm
Particle size distribution:
Particle size distribution of product prepared according to Example 9 is shown in Fig. 19.

**Example 10**

A mixture comprising 20L of 1-hexanol and 200 ml of purified water was prepared in a reactor. 5.00 kg of solifenacin succinate was added and the formed suspension was heated to 85 °C (RT) and maintained at this temperature for the next 30 minutes for all solid to dissolve. Continuous homogenization with mechanical mixer was applied during the crystallization process. Formed clear solution was cooled to 58 °C (RT) in 30 minutes. 50 g of seeds of pure solifenacin were added at the temperature of 58 °C and maintained at this temperature for next 60 minutes. Then the formed suspension was cooled with average cooling ramp 0.28 K/min to 10 °C (in 180 minutes) and maintained at this temperature for the next 30 minutes for the crystallization to complete. Finally, the formed product was isolated with filtration and washed with 2L of 1-hexanol, cooled to 10 °C.
The isolated product was dried in a vacuum drier at 60 °C with a nitrogen flow applied. 4.58 kg of dried product was obtained.

Yield = 92 %

Average particle size: 112 $\mu$m

Particle size distribution:

| $D_{10}$ | 18.6 $\mu$m |
|---|---|
| $D_{50}$ | 77.5 $\mu$m |
| $D_{90}$ | 246.4 $\mu$m |

Particle size distribution of product prepared according to Example 10 is shown in Fig. 20.

**Example 11**

2.00 g solifenacine base (chromatographic purity 93.0 %), 0.68 g succinic acid and 10.6 ml 1-hexanol were charged into the three-necked flask and heated to 85 C. At 50 °C 0.10 ml demineralised water was added to suspension and clear solution is gained. After reaching the 85 °C, solution is cooled in previously defined manner to room temperature, filtered and washed with 1-hexanol. 2.0 g of product were received, with 99.97 % ee purity and 99.26 % chromatographic purity.

**Example 12**

2.95 g solifenacine base (chromatographic purity 97.0 %), 1.01 g succinic acid and 12.5 ml 1-hexanol were charged into the three-necked flask and heated to 85 °C. At 50 °C 0.12 ml demineralised water was added to suspension and clear solution is gained. After reaching the 85 °C, solution is cooled in previously defined manner to room temperature, filtered and washed with 1-hexanol. 2.7 g of product were received, with 99.98 % ee purity and 99.27 % chromatographic purity.

**Example 13**

21.75 g solifenacine base (chromatographic purity 99.0 %, ee purity 99.9 %), 7.44 g succinic acid and 87 ml 1-hexanol were charged into the three-necked flask and heated to 85 C. At 65 °C 0.87 ml demineralised water was added to suspension and clear solution is gained. After reaching the 85 °C, solution is cooled in previously defined manner (seed crystals were added at 65 °C) to room temperature, filtered and washed with 1-hexanol. 21.0 g of product were received, with 99.91 % ee purity.

**Claims**

1.   A crystallization process for the preparation and/or purification of solifenacin succinate applying a mixture of $C_5$-$C_{10}$ alcohols and water.

2.   A process according to claim 1, wherein the solvent mixture used for the crystallization process comprises water and a $C_5$ to $C_{10}$ alcohol selected from the group consisting of n-pentanol, isopentanol, sec-pentanol, n-hexanol, iso hexanol, sec-hexanol, n-heptanol, n-octanol, n-nonanol and n-decanol.

3.   A process according to any of claims 1 and 2, **characterized in that** the alcohol is chosen from the group of n-pentanol and n-hexanol.

4.   A process according to any of claims 1 and 2, **characterized in that**:

i.) A solifenacin salt, which is solifenacin succinate, is dissolved in a solvent mixture comprising water and a $C_5$ to $C_{10}$ alcohol, preferably n-pentanol, n-hexanol, or n-heptanol, most preferably n-hexanol, at a temperature between 40°C and 150°C, preferably between 75°C and 100°C until all solifenacin salt dissolves,
ii.) cooling the dissolved mixture to the seeding temperature $T_{seeding}$, which is between 40°C and 80°C, preferably between 50°C and 70°C, most preferably between 50°C and 60°C,
iii.) optionally seeding the dissolved mixture with crystals of solifenacin salt,

iv.) optionally aging the seeded media to additionally control the nucleation process for 10 to 120 minutes,
v.) cooling the mixture to a final temperature between -40 °C and 40 °C, preferably to a temperature between 5°C and 10°C in a time between 0.5 and 10 hours, preferably in between 2 and 3 hours, and maintained at the final temperature for 15 minutes to 20 hours if needed,
vi.) filtering and washing the crystals obtained.

5. A process according to any of claims 1 and 2, **characterized in that**:

i.) solifenacin base, is dissolved in a solvent mixture comprising a $C_5$ to $C_{10}$ alcohol, preferably n-pentanol, n-hexanol, or n-heptanol, most preferably n-hexanol, and comprising water, at a any given temperature,
ii.) addition of the counter acid, which can be in concentrated form (solid, gas or liquid) or separately dissolved or suspended in any (anti)solvent,
iii.) optionally seeding the dissolved mixture with crystals of solifenacin salt at the seeding temperature $T_{seeding}$ between 40°C and 80°C, preferably between 50°C and 70°C, most preferably between 50°C and 60°C,
iv.) optionally aging the seeded media,
v.) cooling the mixture to a final temperature between -40 °C and 40 °C, in a time between 0.5 and 10 hours, preferably in between 2 and 3 hours, and maintaining it at the final temperature for 15 minutes to 20 hours if needed,
vi.) filtering and washing the crystals obtained.

6. A process according to any of claims 1 to 5, **characterized in that** the cooling ramp to the final temperature is between 0.1 K/min to 1.5 K/min, preferably between 0.2 K/min and 0.5 K/min.

7. A process according to any of claims 1 to 6, **characterized in that** the mass to volume ratio between solifenacin succinate, and the solvent mixture applied is between 1/50 to 1/1 kg/L, preferably between 1/10 to 1/1.5 kg/L and most preferably between 1/5 to 1/2 kg/L.

8. A process according to any of claims 1 to 7, **characterized in that** after the supersaturated media is seeded, the aging at the temperature of seeding is applied.

9. A process according to any of claims 1 to 8, **characterized in that** the temperature of seeding is below 65 °C.

10. A crystallization process for solifenacin succinate according to any of the preceding claims, using alcohol/water mixtures **characterized in** seeding and/or aging below 65 °C.

11. A process according to any of claims 1 to 9, **characterized in that** material with one-modal particle size distribution is obtained.

12. A process according to any of claims 1 to 10, **characterized in that** material with one-modal particle size distribution and average particle size between 40 - 250 $\mu$m is obtained.

13. A process according to any of claims 1 to 12, **characterized in that** the crystallisation process for the preparation and/or purification of solifenacin succinate applies a mixture of at least one $C_5$-$C_{10}$ alcohol and at least one further alcohol having less than 5 or more than 10 carbon atoms, with water.

**Patentansprüche**

1. Kristallisationsverfahren zur Herstellung und/oder Aufreinigung von Solifenacinsuccinat, bei dem eine Mischung von $C_5$-$C_{10}$-Alkoholen und Wasser eingesetzt wird.

2. Verfahren nach Anspruch 1, wobei die für das Kristallisationsverfahren verwendete Lösungsmittelmischung Wasser und einen $C_5$-$C_{10}$-Alkohol, ausgewählt aus der Gruppe bestehend aus n-Pentanol, Isopentanol, sek.-Pentanol, n-Hexanol, Iso-Hexanol, sek.-Hexanol, n-Heptanol, n-Octanol, n-Nonanol und n-Decanol, umfasst.

3. Verfahren nach einem der Ansprüche 1 und 2, **dadurch gekennzeichnet, dass** der Alkohol aus der Gruppe n-Pentanol und n-Hexanol ausgewählt wird.

**4.** Verfahren nach einem der Ansprüche 1 und 2, **dadurch gekennzeichnet, dass** man:

i) ein Solifenacinsalz, bei dem es sich um Solifenacinsuccinat handelt, in einer Lösungsmittelmischung umfassend Wasser und einen $C_5$-$C_{10}$-Alkohol, vorzugsweise n-Pentanol, n-Hexanol oder n-Heptanol, am stärksten bevorzugt n-Hexanol, bei einer Temperatur zwischen 40°C und 150°C, vorzugsweise zwischen 75°C und 100°C, löst, bis das Solifenacinsalz vollständig gelöst ist,

ii) die gelöste Mischung auf die Animpftemperatur $T_{Animpfen}$, die zwischen 40°C und 80°C, vorzugsweise zwischen 50°C und 70°C, am stärksten bevorzugt zwischen 50°C und 60°C, beträgt, abkühlt,

iii) gegebenenfalls die gelöste Mischung mit Solifenacinsalzkristallen animpft,

iv) gegebenenfalls das angeimpfte Medium reifen lässt, um den Kristallkernbildungsvorgang zusätzlich 10 bis 120 Minuten lang zu kontrollieren,

v) die Mischung auf eine Endtemperatur zwischen -40°C und 40°C, vorzugsweise auf eine Temperatur zwischen 5°C und 10°C, abkühlt, und zwar über einen Zeitraum von zwischen 0,5 und 10 Stunden, vorzugsweise zwischen 2 und 3 Stunden, und gegebenenfalls 15 Minuten bis 20 Stunden bei der Endtemperatur hält,

vi) die erhaltenen Kristalle abfiltriert und wäscht.

**5.** Verfahren nach einem der Ansprüche 1 und 2, **dadurch gekennzeichnet, dass** man

i) Solifenacin-Base in einer Lösungsmittelmischung, umfassend einen $C_5$-$C_{10}$-Alkohol, vorzugsweise n-Pentanol, n-Hexanol oder n-Heptanol, am stärksten bevorzugt n-Hexanol, und Wasser, bei einer bestimmten Temperatur löst,

ii) die Gegensäure, die in konzentrierter Form (Feststoff, Gas oder Flüssigkeit) oder getrennt in einem beliebigen (anti-)Lösungsmittel gelöst oder suspendiert sein kann, zugibt,

iii) gegebenenfalls die gelöste Mischung mit Solifenacinsalzkristallen bei der Animpftemperatur $T_{Animpfen}$ von zwischen 40°C und 80°C, vorzugsweise zwischen 50°C und 70°C, am stärksten bevorzugt zwischen 50°C und 60°C, animpft,

iv) gegebenenfalls das angeimpfte Medium reifen lässt,

v) die Mischung auf eine Endtemperatur zwischen -40°C und 40°C abkühlt, und zwar über einen Zeitraum von zwischen 0,5 und 10 Stunden, vorzugsweise zwischen 2 und 3 Stunden, und gegebenenfalls 15 Minuten bis 20 Stunden bei der Endtemperatur hält,

vi) die erhaltenen Kristalle abfiltriert und wäscht.

**6.** Verfahren nach einem der Ansprüche 1 bis 5, **dadurch gekennzeichnet, dass** das Hinabfahren auf die Endtemperatur zwischen 0,1 K/min und 1,5 K/min, vorzugsweise zwischen 0,2 K/min und 0,5 K/min, erfolgt.

**7.** Verfahren nach einem der Ansprüche 1 bis 6, **dadurch gekennzeichnet, dass** das Masse-VolumenVerhältnis zwischen Solifenacinsuccinat und der eingesetzten Lösungsmittelmischung zwischen 1/50 und 1/1 kg/l, vorzugsweise zwischen 1/10 und 1/1,5 kg/l und am stärksten bevorzugt zwischen 1/5 und 1/2 kg/l beträgt.

**8.** Verfahren nach einem der Ansprüche 1 bis 7, **dadurch gekennzeichnet, dass** nach dem Animpfen des übersättigten Mediums bei der Animpftemperatur reifen gelassen wird.

**9.** Verfahren nach einem der Ansprüche 1 bis 8, **dadurch gekennzeichnet, dass** die Animpftemperatur unter 65°C beträgt.

**10.** Kristallisationsverfahren für Solifenacinsuccinat nach einem der vorhergehenden Ansprüche unter Verwendung von Alkohol-Wasser-Mischungen, **dadurch gekennzeichnet, dass** unter 65°C angeimpft und/oder gereift wird.

**11.** Verfahren nach einem der Ansprüche 1 bis 9, **dadurch gekennzeichnet, dass** ein Material mit einer einmodalen Teilchengrößenverteilung erhalten wird.

**12.** Verfahren nach einem der Ansprüche 1 bis 10, **dadurch gekennzeichnet, dass** ein Material mit einer einmodalen Teilchengrößenverteilung und einer durchschnittlichen Teilchengröße zwischen 40-250 $\mu$m erhalten wird.

**13.** Verfahren nach einem der Ansprüche 1 bis 12, **dadurch gekennzeichnet, dass** bei dem Kristallisationsverfahren zur Herstellung und/oder Aufreinigung von Solifenacinsuccinat eine Mischung von mindestens einem $C_5$-$C_{10}$-Alkohol und mindestens einem weiteren Alkohol mit weniger als 5 oder mehr als 10 Kohlenstoffatomen mit Wasser eingesetzt wird.

**Revendications**

1. Un procédé de cristallisation pour la préparation et/ou la purification de succinate de solifénacine, en mettant en oeuvre un mélange d'alcools en $C_5$-$C_{10}$ et d'eau.

2. Un procédé selon la revendication 1, dans lequel le mélange de solvants utilisé pour le procédé de cristallisation comprend de l'eau et un alcool en $C_5$ à $C_{10}$ choisi parmi le groupe constitué du n-pentanol, de l'isopentanol, du sec-pentanol, du n-hexanol, l'iso hexanol, du sec-hexanol, du n-heptanol, du n-octanol, du n-nonanol et du n-décanol.

3. Un procédé selon l'une quelconque des revendications 1 et 2, **caractérisé en ce que** l'alcool est choisi parmi le groupe du n-pentanol et du n-hexanol.

4. Un procédé selon l'une quelconque des revendications 1 et 2, **caractérisé en ce que** :

   i) un sel de solifénacine, qui est du succinate de solifénacine, est dissous dans un mélange de solvants comprenant de l'eau et un alcool en $C_5$ à $C_{10}$, de préférence le n-pentanol, le n-hexanol ou le n-heptanol, avec la plus grande préférence le n-hexanol, à une température comprise entre 40 °C et 150 °C, de préférence entre 75 °C et 100 °C, jusqu'à ce que tout le sel de solifénacine soit dissous,
   (ii) refroidissement du mélange dissous à la température d'ensemencement $T_{ensemencement}$, qui est comprise entre 40 °C et 80 °C, de préférence entre 50 °C et 70 °C, avec la plus grande préférence entre 50 °C et 60 °C,
   (iii) facultativement, ensemencement du mélange dissous avec des cristaux de sel de solifénacine,
   iv) facultativement, vieillissement du milieu ensemencé afin de réguler davantage le processus de nucléation pendant 10 à 120 minutes,
   v) refroidissement du mélange à une température finale comprise entre -40 °C et 40 °C, de préférence à une température comprise entre 5 °C et 10 °C pendant un laps de temps compris entre 0,5 et 10 heures, de préférence entre 2 et 3 heures, et maintien de celui-ci à la température finale pendant 15 minutes à 20 heures si nécessaire,
   vi) filtration et lavage des cristaux obtenus.

5. Un procédé selon l'une quelconque des revendications 1 et 2, **caractérisé en ce que** :

   i) la solifénacine base est dissoute dans un mélange de solvants comprenant un alcool en $C_5$ à $C_{10}$, de préférence le n-pentanol, le n-hexanol ou le n-heptanol, avec la plus grande préférence le n-hexanol, et comprenant de l'eau, à n'importe quelle température donnée,
   ii) addition de l'anti-acide, qui peut être sous forme concentrée (solide, gazeuse ou liquide) ou dissous ou mis en suspension séparément dans n'importe quel (anti-)solvant,
   iii) facultativement, ensemencement du mélange dissous avec des cristaux de solifénacine à la température d'ensemencement $T_{ensemencement}$, comprise entre 40 °C et 80 °C, de préférence entre 50 °C et 70 °C, avec la plus grande préférence entre 50 °C et 60 °C,
   iv) facultativement, vieillissement du milieu ensemencé,
   v) refroidissement du mélange à une température finale comprise entre -40 °C et 40 °C, pendant un laps de temps compris entre 0,5 et 10 heures, de préférence entre 2 et 3 heures, et maintien de celui-ci à la température finale pendant 15 minutes à 20 heures si nécessaire,
   vi) filtration et lavage des cristaux obtenus.

6. Un procédé selon l'une quelconque des revendications 1 à 5, **caractérisé en ce que** la rampe de refroidissement jusqu'à la température finale est comprise entre 0,1 K/min et 1,5 K/min, de préférence entre 0,2 K/min et 0,5 K/min.

7. Un procédé selon l'une quelconque des revendications 1 à 6, **caractérisé en ce que** le rapport masse/volume entre le succinate de solifénacine et le mélange de solvants appliqué est compris entre 1/50 et 1/1 kg/l, de préférence entre 1/10 et 1/1,5 kg/l et avec la plus grande préférence, entre 1/5 et 1/2 kg/l.

8. Un procédé selon l'une quelconque des revendications 1 à 7, **caractérisé en ce qu'**après l'ensemencement du milieu supersaturé, le vieillissement à la température d'ensemencement est appliqué.

9. Un procédé selon l'une quelconque des revendications 1 à 8, **caractérisé en ce que** la température d'ensemencement est inférieure à 65 °C.

10. Un procédé de cristallisation pour le succinate de solifénacine selon l'une quelconque des revendications précédentes, en utilisant des mélanges d'alcool et d'eau, **caractérisé par** un ensemencement et/ou un vieillissement endeçà de 65 °C.

11. Un procédé selon l'une quelconque des revendications 1 à 9, **caractérisé en ce que** l'on obtient une matière présentant une distribution granulométrique monomodale.

12. Un procédé selon l'une quelconque des revendications 1 à 10, **caractérisé en ce que** l'on obtient une matière présentant une distribution granulométrique monomodale et une taille de particule moyenne comprise entre 40 et 250 $\mu$m.

13. Un procédé selon l'une quelconque des revendications 1 à 12, **caractérisé en ce que** le procédé de cristallisation pour la préparation et/ou la purification du succinate de solifénacine met en oeuvre un mélange d'au moins un alcool en $C_5$-$C_{10}$ et d'au moins un autre alcool comportant moins de 5 ou plus de 10 atomes de carbone, et d'eau.

## Figure 1

**Crystals of solifenacin succinate, prepared according to Example 1**

## Figure 2

**Crystals of solifenacin succinate, prepared according to Example 2**

**Figure 3**

**Crystals of solifenacin succinate, prepared according to Example 3**

Figure 4

Crystals of solifenacin succinate, prepared according to Example 4

## Figure 5

## Crystals of solifenacin succinate, prepared according to Example 5

Figure 6

Crystals of solifenacin succinate, prepared according to Example 6

Figure 7

**Crystals of solifenacin succinate, prepared according to Example 7**

**Figure 8**

**Crystals of solifenacin succinate, prepared according to Example 8**

Figure 9

**Crystals of solifenacin succinate, prepared according to Example 9**

Figure 10

**Crystals of solifenacin succinate, prepared according to Example 10**

**Figure 11**

Particle size distribution of product prepared according to Example 1

**Figure 12**

Particle size distribution of product prepared according to Example 2

**Figure 13**

Particle size distribution of product prepared according to Example 3

**Figure 14**

Particle size distribution of product prepared according to Example 4

**Figure 15**

Particle size distribution of product prepared according to Example 5

**Figure 16**

Particle size distribution of product prepared according to Example 6

## Figure 17

Particle size distribution of product prepared according to Example 7

## Figure 18

Particle size distribution of product prepared according to Example 8

**Figure 19**

Particle size distribution of product prepared according to Example 9

**Figure 20**

Particle size distribution of product prepared according to Example 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1714965 A1
- WO 2008011462 A2
- WO 2008013851 A